(19) Europäisches Patentamt
European Patent Office
Office européen des brevets

(11) EP 4 304 622 B1

(12) # FASCICULE DE BREVET EUROPEEN

(45) Date de publication et mention de la délivrance du brevet:
**14.01.2026 Bulletin 2026/03**

(21) Numéro de dépôt: **22714228.8**

(22) Date de dépôt: **10.03.2022**

(51) Classification Internationale des Brevets (IPC):
***A61K 36/37*** *(2006.01)* ***A61K 36/61*** *(2006.01)*
***A61K 8/9789*** *(2017.01)* ***A61Q 19/00*** *(2006.01)*
***A61P 17/10*** *(2006.01)*

(52) Classification Coopérative des Brevets (CPC):
(C-Sets disponibles)
**A61P 17/10; A61K 8/9789; A61K 36/37;**
**A61K 36/61; A61Q 19/00;** A61K 2236/35;
A61K 2236/39; Y02A 50/30 (Cont.)

(86) Numéro de dépôt international:
**PCT/FR2022/050431**

(87) Numéro de publication internationale:
**WO 2022/189758 (15.09.2022 Gazette 2022/37)**

(54) **ASSOCIATION D'UN EXTRAIT DE MYRTE ET D'UN EXTRAIT DE TRIPTERYGIUM WILFORDII POUR LUTTER CONTRE L'INFLAMMATION INDUITE PAR C. ACNES**

KOMBINATION AUS EINEM MYRTEEXTRAKT UND EINEM TRIPTERYGIUM WILFORDII-EXTRAKT ZUR BEKÄMPFUNG VON C. ACNES-INDUZIERTER ENTZÜNDUNG

COMBINATION OF A MYRTLE EXTRACT AND A TRIPTERYGIUM WILFORDII EXTRACT FOR CONTROLLING C. ACNES-INDUCED INFLAMMATION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **10.03.2021 FR 2102343**

(43) Date de publication de la demande:
**17.01.2024 Bulletin 2024/03**

(73) Titulaire: **Pierre Fabre Dermo-Cosmétique**
**81500 Lavaur (FR)**

(72) Inventeurs:
- **BESSOU-TOUYA, Sandrine**
**81106 Castres (FR)**
- **GARIDOU, Lucile**
**81106 Castres (FR)**
- **MIAS, Céline**
**31320 Auzeville Tolosan (FR)**

(74) Mandataire: **Regimbeau**
**20, rue de Chazelles**
**75847 Paris Cedex 17 (FR)**

(56) Documents cités:
**CA-A1- 3 023 192 FR-A1- 2 783 425**
**FR-A1- 2 992 862**

- **ROTSTEIN A ET AL: "ISOLATION AND ANTIBACTERIAL ACTIVITY OF ACYLPHLOROGLUCINOLS FROM MYRTUS COMMUNIS", ANTIMICROBIAL AGENTS AND CHEMOTHERAPY, AMERICAN SOCIETY FOR MICROBIOLOGY, US, vol. 6, no. 5, 1 November 1974 (1974-11-01), pages 539 - 542, XP002105891, ISSN: 0066-4804**
- **DUAN H ET AL: "Triterpenoids from Tripterygium wilfordii", PHYTOCHEMISTRY, ELSEVIER, AMSTERDAM , NL, vol. 53, no. 7, 1 April 2000 (2000-04-01), pages 805 - 810, XP004291375, ISSN: 0031-9422, DOI: 10.1016/S0031-9422(00) 00007-8**
- **BASAK S A ET AL: "Acne and its management", PEDIATRICS IN REVIEW, AMERICAN ACADEMY OF PEDIATRICS, vol. 34, no. 11, 1 January 2013 (2013-01-01), pages 479 - 497, XP009184555, ISSN: 0191-9601, DOI: 10.1542/PIR.34-11-479**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

(52) Classification Coopérative des Brevets (CPC):
(Cont.)

C-Sets
**A61K 36/37, A61K 2300/00;**
**A61K 36/61, A61K 2300/00**

## Description

### DOMAINE TECHNIQUE DE L'INVENTION

La présente invention concerne une nouvelle association comprenant un extrait de myrte et un extrait de *Tripterygium wilfordii,* ainsi que des compositions comprenant cette association, en particulier pour leur utilisation dans le domaine de l'acné, et notamment dans le traitement de l'inflammation induite par *C. acnes,* telles que définies dans les revendications.

### ETAT DE LA TECHNIQUE

L'acné est une affection cutanée courante et multifactorielle des follicules pileux et des glandes sébacées entrainant une formation de comédons, touchant le visage, la région scapulaire, les bras et les régions intertrigineuses. Elle est la principale cause des dermatoses les plus fréquentes. Il est important de ne pas banaliser cette affection et de la traiter correctement car elle peut avoir des conséquences psychosociales invalidantes notamment du fait de la formation de cicatrices.

Il existe plusieurs formes d'acné, le facteur commun de l'ensemble d'entre elles étant l'attaque des follicules pilosébacés. On peut citer par exemple, l'acné commune, l'acné conglobata, l'acné chéloïde de la nuque, l'acné médicamenteuse, l'acné miliaire récurrente, l'acné nécrotique, l'acné néonatale, l'acné prémenstruelle, l'acné professionnelle, l'acné sénile et l'acné solaire.

L'acné commune ou acné vulgaire, également appelée acné polymorphe juvénile, est la plus courante et comprend quatre stades :

- Le stade 1 correspond à l'acné comédonnienne et est caractérisé par un grand nombre de comédons ouverts et/ou fermés et de microkystes ;
- Le stade 2, ou acné papulo-pustuleuse, est de gravité légère à modérée et est caractérisé par la présence de comédons ouverts et/ou fermés, de microkystes, mais également de papules et pustules rouges. Il affecte principalement le visage et laisse quelques cicatrices ;
- Le stade 3, ou acné papulo-comédonnienne, est plus grave et s'étend au dos, au thorax et aux épaules. Il est accompagné d'un grand nombre de cicatrices ;
- Le stade 4, ou acné nodulo-kystique, est accompagné de nombreuses cicatrices. Il présente des nodules et également des pustules pourpres volumineux et douloureux.

Sous la forme la plus légère, l'acné concerne presque chaque être humain. Sa fréquence est maximale à l'âge de la puberté, mais elle peut se manifester pour la première fois dès l'âge de 7 à 9 ans et jusqu' à des âges dépassant 40 ans. Il est fréquent de souffrir d'acné encore après 25 ans. L'acné touche en outre aussi bien les hommes que les femmes. Au cours de la puberté, sous l'influence des sécrétions hormonales et en particulier des androgènes, mais aussi associé à différents facteurs externes, il est observé une surproduction de sébum appelée hyperséborrhée. Chez des sujets prédisposés à l'acné, cet environnement est propice au développement de la bactérie clé de l'acné, *Cutibacterium acnes* (*C. acnes*) (anciennement appelée *Propionibacterium acnes*). Cette bactérie métabolise les triglycérides cutanés en acide gras irritants *via* des lipases qui agressent la paroi du follicule et du derme environnant, produit également divers enzymes et chimioattractants des cellules phagocytaires de l'immunité, et stimule la production de cytokines pro-inflammatoires par différents types de cellules (sébocytes, kératinocytes, monocytes, notamment) qui aggravent l'inflammation. On a longtemps pensé que la lutte contre cette espèce bactérienne était prioritaire dans le traitement de l'acné. L'usage de divers antimicrobiens topiques sont aujourd'hui encore largement utilisés (peroxyde de benzoyle, érythromycine, triclosan). Mais depuis peu, les chercheurs ont compris que le but n'est pas d'éradiquer *C. acnes* qui est une bactérie commensale, nécessaire à l'homéostasie tissulaire, mais de rétablir un équilibre car l'acné est associée à une perte de la diversité des phylotypes de *C. acnes* avec une prédominance du phylotype pathogène IA1. Contrairement à ce que l'on a longtemps pensé, l'acné n'est pas associée à une multiplication de *C. acnes* mais à une modification du ratio des phylotypes et une perte de la richesse en ces phylotypes.

De même, une antibiothérapie systémique plus ou moins prolongée était parfois associée au traitement selon la gravité de l'affection (tétracyclines, doxycycline). Les dermatologues vont aujourd'hui plus se tourner vers des anti-inflammatoires locaux, séborégulateurs.

On a constaté des échecs fréquents à tous ces traitements, dus souvent à une forte proportion de souches résistantes de *C. acnes.* Cette résistance peut être la conséquence d'une organisation des populations bactériennes en biofilm. Les biofilms sont des communautés cellulaires bactériennes intégrées dans une matrice extracellulaire excrétée par les microorganismes, composée de polymères de sucres et appelée glycocalyx. Les bactéries sessiles (associées au biofilm) sont phénotypiquement et physiologiquement différentes des bactéries planctoniques (libres). Des études ont

confirmé l'aptitude de *C. acnes* à former des biofilms aussi bien *in vitro* (Holmberg et al., Clin. Microbiol. Infect. 2009, 15, 787-795) qu'*in vivo* sur des dispositifs médicaux (Craig et al., J. Am. Acad. Dermatol. 2007, 722-724).

Cette bactérie joue un rôle pivot dans l'acné, notamment en stimulant la réponse inflammatoire locale (Dagnelie et al., Journal of the European Academy of Dermatology 2019, 33(12), 2340-2348). Contrairement à ce que l'on a longtemps cru, *C. acnes* ne prolifère pas dans le follicule pilo-sébacé des peaux acnéiques mais on observe une perte de la richesse/diversité des différents phylotypes de cette bactérie avec une forte prédominance du phylotype IA1. Ce phylotype est pro-pathogène et possède une forte capacité à s'organiser en biofilm conférant une plus forte virulence à cette bactérie. Cette virulence peut être mesurée par la quantification des facteurs de virulence produits par *C. acnes.*

*C. acnes* IA1 stimule la réponse inflammatoire locale en agissant directement sur les cellules du follicule pilo-sébacé et notamment les kératinocytes, les sébocytes mais aussi les cellules immunitaires : les monocytes. Elle agit en stimulant la production de cytokines pro-inflammatoires par ces cellules et notamment les interleukines 6 et 8. Il s'en suit une cascade immuno-inflammatoire et plus particulièrement le recrutement et la différenciation de lymphocytes T CD4+ naïfs en LTH17. Ces LTH17, nouvellement mis en évidence dans la pathologie de l'acné, produisent spécifiquement l'interleukine 17 dès les stades infracliniques de l'acné jusqu'aux lésions les plus inflammatoires.

Ainsi, il existe toujours un besoin de fournir des traitements plus efficaces de l'acné ne présentant pas d'effets indésirables chez le patient. En particulier, il n'existe pas à ce jour d'agents qui agissent à la fois directement sur le biofilm de *C. acnes* et sur les réactions inflammatoires qui en résultent.

## RESUME DE L'INVENTION

La présente invention a pour objet de répondre à ces besoins. En effet, les inventeurs ont mis en évidence, de façon tout à fait inattendue, que l'association d'un extrait de myrte et d'un extrait de *Tripterygium wilfordii* avaient la capacité de réduire la production de cytokines inflammatoires, l'inflammation étant stimulée en présence de C. *acnes.* En effet, l'association selon l'invention présente l'avantage d'agir de façon synergique sur la cascade immuno-inflammatoire médiée par *C. acnes* et d'être ainsi utile dans le traitement de l'acné.

La présente invention a donc pour objet une association telle que définie dans la revendication 1, comprenant un extrait de myrte et un extrait de *Tripterygium wilfordii,.*

La présente invention a également pour objet une composition cosmétique ou dermatologique telle que définie dans la revendication 11, comprenant une association comprenant un extrait de myrte et un extrait de *Tripterygium wilfordii,* avec au moins un excipient cosmétiquement ou dermatologiquement acceptable,

La présente invention a également pour objet une association selon l'invention, c'est-à-dire comprenant un extrait de myrte et un extrait de *Tripterygium wilfordii,* pour son utilisation dans le traitement de l'inflammation induite par C. *acnes,* telle que définie dans la revendication 9. L'invention concerne également l'utilisation d'une association selon l'invention pour la préparation d'une composition cosmétique ou dermatologique destinée au traitement de l'inflammation induite par *C. acnes.*

L'invention concerne également l'utilisation d'une association selon l'invention dans le traitement de l'inflammation induite par *C. acnes.*

L'invention concerne également une méthode de traitement de l'inflammation induite par *C. acnes* comprenant l'administration à une personne en ayant besoin d'une quantité efficace d'une association selon l'invention.

La présente invention a également pour objet une association selon l'invention pour son utilisation dans le traitement de l'acné ou d'une peau acnéique, telle que définie dans la revendication 10.

L'invention concerne également l'utilisation d'une association selon l'invention pour la préparation d'une composition cosmétique ou dermatologique destinée au traitement de l'acné ou d'une peau acnéique.

L'invention concerne également l'utilisation d'une association selon l'invention dans le traitement de l'acné ou d'une peau acnéique.

L'invention concerne également une méthode de traitement de l'acné ou d'une peau acnéique comprenant l'administration à une personne en ayant besoin d'une quantité efficace d'une association selon l'invention.

La présente invention a également pour objet une composition cosmétique ou dermatologique selon l'invention telle que définie dans la revendication 15, c'est-à-dire comprenant une association comprenant un extrait de myrte et un extrait de *Tripterygium wilfordii,* avec au moins un excipient cosmétiquement ou dermatologiquement acceptable, pour son utilisation dans le traitement de l'inflammation induite par *C. acnes.*

L'invention concerne également l'utilisation d'une composition cosmétique ou dermatologique selon l'invention pour la préparation d'un médicament destiné au traitement de l'inflammation induite par *C. acnes.*

L'invention concerne également l'utilisation d'une composition cosmétique ou dermatologique selon l'invention dans le traitement de l'inflammation induite par *C. acnes.*

L'invention concerne également une méthode de traitement de l'inflammation induite par *C. acnes* comprenant l'administration à une personne en ayant besoin d'une quantité efficace d'une composition cosmétique ou dermatologique selon l'invention.

La présente invention a également pour objet une composition cosmétique ou dermatologique selon l'invention telle que définie dans la revendication 16, pour son utilisation dans le traitement de l'acné ou d'une peau acnéique.
L'invention concerne également l'utilisation d'une composition cosmétique ou dermatologique selon l'invention pour la préparation d'un médicament destiné au traitement de l'acné ou d'une peau acnéique.
L'invention concerne également l'utilisation d'une composition cosmétique ou dermatologique selon l'invention dans le traitement de l'acné ou d'une peau acnéique.
L'invention concerne également une méthode de traitement de l'acné ou d'une peau acnéique comprenant l'administration à une personne en ayant besoin d'une quantité efficace d'une composition cosmétique ou dermatologique selon l'invention.

## DESCRIPTION DETAILLEE DE L'INVENTION

Selon un premier aspect, l'invention concerne une association comprenant un extrait de myrte et un extrait de *Tripterygium wilfordii.*

<u>Extrait de myrte</u>

Le myrte commun, *Myrtus communis* L., est un arbuste de la famille des Myrtaceae. L'extrait de myrte selon la présente invention est réalisé plus particulièrement à partir des feuilles de *Myrtus communis.* De préférence, il s'agit d'une fraction apolaire de parties aériennes, et plus particulièrement des feuilles, de myrte.
Par « faction apolaire », on entend une fraction d'un extrait apolaire, typiquement un extrait apolaire traité par du charbon actif de sorte à éliminer des chlorophylles.
Par « extrait apolaire », on entend un extrait susceptible d'être obtenu à l'aide d'un solvant d'extraction apolaire, tel que par exemple l'acétate d'éthyle, l'acétate d'isopropyle ou un mélange de ceux-ci.
Par « parties aériennes » selon l'invention, on entend les parties de la plante situées au-dessus du sol, par exemple, les feuilles, les tiges, les pétioles et/ou les inflorescences, notamment les feuilles.
L'extrait selon la présente invention est susceptible d'être obtenu par extraction à l'aide d'un solvant ou mélange de solvants (appelé solvant d'extraction) choisi parmi :

- les alcools tels que l'éthanol, le méthanol, l'isopropanol,
- les cétones dont l'acétone et la méthyléthylacétone,
- l'hexane,
- le chlorure de méthylène,
- l'éther isopropylique,
- l'acétate d'éthyle ou d'isopropyle,
- et leurs mélanges ;

ou par extraction à l'aide de $CO_2$ supercritique.
Selon un mode de réalisation, l'extrait de myrte est susceptible d'être obtenu par extraction à l'aide d'acétate d'isopropyle, de préférence à partir des parties aériennes, et en particulier des feuilles, de *Myrtus communis.*
Ainsi, la plante (ou toute partie de celle-ci) est mise en contact avec le solvant d'extraction. Une fois l'extraction effectuée, le mélange plante-solvant d'extraction est filtré de sorte à séparer la phase solvant des résidus de plante (appelés marc). Le marc ainsi obtenu est rincé, typiquement avec le même solvant que le solvant d'extraction, et le solvant de rinçage ainsi obtenu est mélangé avec la phase solvant de sorte à obtenir un jus d'extraction.
Avantageusement, les jus d'extraction, obtenus après filtration et rinçage du marc, sont décolorés par addition de charbon actif, ce qui permet l'élimination des chlorophylles.
L'extrait peut également être stabilisé par addition d'un antioxydant comme par exemple le butylhydroxytoluène ou l'alpha tocophérol, notamment en des quantités comprises entre 0,05 et 1 % en poids d'extrait sec.
L'extrait selon la présente invention présente notamment la caractéristique de comprendre, et notamment d'être riche en, myrtucommulones et acide ursolique. Les myrtucommulones A, B', D, B, isos (isosemimyrtucommulmone) et S (semimyrtucommulone) sont avantageusement présentes dans l'extrait et sont notamment les principales myrtucommulones présentes.
Avantageusement, la teneur en myrtucommulones totales dans l'extrait est comprise entre 3 et 10 % en poids d'extrait sec.
La teneur en acide ursolique est comprise entre 10 et 30%, de préférence $\geq$ 15% en poids d'extrait sec.
Ces molécules portent au moins en partie l'activité revendiquée dans le cadre de la présente invention.
Dans un mode de réalisation particulier de l'invention, l'extrait de myrte sera de préférence tel que décrit dans la demande de brevet EP 1 112 079, ou selon l'exemple 1 de la présente demande. Ce document EP 1 112 079 décrit les

propriétés antibactériennes de l'extrait de *Myrtus communis* et ses applications dans des compositions cosmétiques ou dermatologiques.

L'extrait de myrte tel qu'utilisé dans la présente invention a également fait l'objet du brevet FR 2 992 862, son activité anti-biofilm vis-à-vis de *C. acnes* y étant décrite.

Extrait de *Tripterygium wilfordii*

*Tripterygium wilfordii* est une plante médicinale appartenant à la famille des Celastraceae. Les terpènes sont parmi les composants les plus actifs de la plante et sont localisés majoritairement dans les racines de la plante. On trouve notamment les triterpènes pentacycliques, tels que la Tingénine A (encore appelée Tingénone ou Mayténine), la Tingénine B (encore appelée 22béta-hydroxy-tingénone), le Célastrol, la Pristimérine et la Tripterygone. Ces molécules sont décrites dans la demande de brevet EP 3 454 875.

L'extrait de *Tripterygium wilfordii* est un extrait comprenant, notamment enrichi en, triterpène(s) pentacyclique(s), tels que Tingénine A, Tingénine B et/ou Célastrol. L'extrait peut comprendre éventuellement des composants (dont des composants actifs) issus de la plante autres que des triterpènes pentacycliques.

Par « triterpène pentacyclique » selon l'invention, on entend un triterpène pentacyclique naturellement produit par les cellules de la plante *Tripterygium wilfordii* et notamment le Célastrol de formule Chem. I, la Tingénine A (encore appelée Tingénone ou Mayténine) de formule Chem. II, la Tingénine B (encore appelée 22béta-Hydroxy-tingénone) de formule Chem. III, la Pristimérine de formule Chem. IV, et/ou la Tripterygone de formule Chem. V, de préférence le Célastrol, la Tingénine A et/ou la Tingénine B, notamment le Célastrol.

[Chem. I]

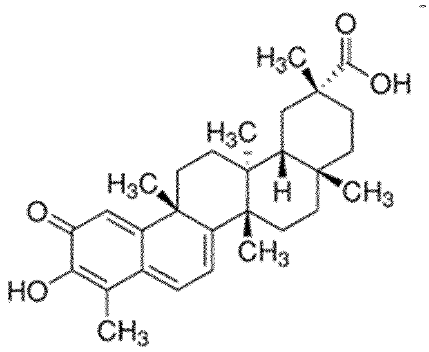

[Chem. II]

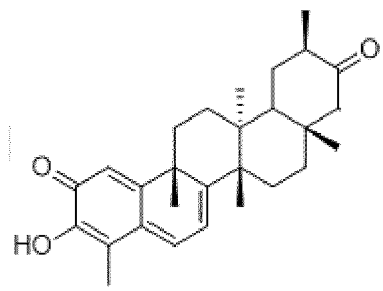

[Chem. III]

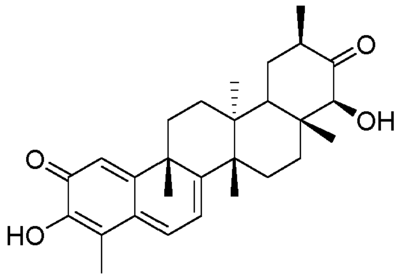

[Chem. IV]

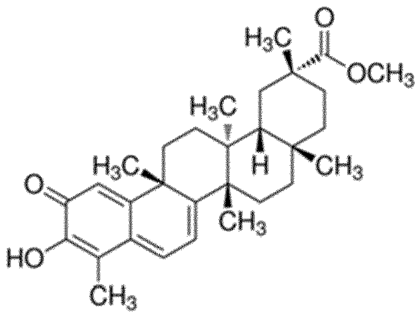

[Chem. V]

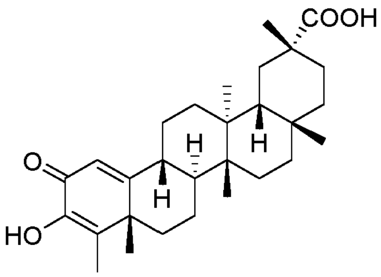

Par « extrait brut », on entend un extrait directement obtenu à partir d'une plante, en l'occurrence *Tripterygium wilfordii.*

Par « extrait enrichi » de *Tripterygium wilfordii,* on entend un extrait de *Tripterygium wilfordii* dans lequel la quantité en triterpène(s) pentacyclique(s), notamment en Tingénine A, Tingénine B et/ou Célastrol, est supérieure à 30% en poids, notamment supérieure à 50% en poids par rapport à la quantité en triterpènes pentacycliques dans un extrait brut sec.

Dans un mode de réalisation, l'extrait, notamment l'extrait enrichi, de *Tripterygium wilfordii* pour une utilisation selon l'invention comprend entre 90% et 100% de triterpène(s) pentacyclique(s) en poids par rapport au poids total de l'extrait sec, notamment de l'extrait enrichi sec.

Les extrait, notamment les extraits bruts ou enrichis, peuvent être obtenus à partir de n'importe quelle partie de la plante *Tripterygium wilfordii,* notamment les racines, les graines ou les parties aériennes.

Alternativement, l'extrait de *Tripterygium wilfordii* peut être obtenu par des cultures de cellules végétales de ces plantes. Dans un tel cas, l'extrait pourra notamment être obtenu à partir du surnageant, de la suspension ou de la biomasse desdites cultures cellulaires, tel que notamment décrit par Coppede et al., Plant Cell Tiss Organ Cult, 2017, 118, 33-43.

Dans un mode de réalisation préféré, l'extrait de *Tripterygium wilfordii* est un extrait comprenant, notamment enrichi en, triterpène(s) pentacyclique(s), et susceptible d'être obtenu selon le procédé suivant :

(i) Une phase de prolifération de cellules de *Tripterygium wilfordii* dans un milieu de prolifération,
(ii) Une phase d'élicitation par ajout d'un cocktail d'élicitation à la culture cellulaire obtenue à l'étape (i), ledit cocktail d'élicitation comprenant au moins un éliciteur de type composé monocarboxylique et au moins un éliciteur biotique, et
(iii) La préparation d'un extrait comprenant, notamment enrichi en, triterpène(s) pentacyclique(s) à partir de la culture

cellulaire obtenue à l'étape (ii).

Par « cellules de *Tripterygium wilfordii »* selon l'invention, on entend les cellules de n'importe quelle partie de la plante : graines, racines, parties aériennes, et notamment des parties aériennes, et plus particulièrement des feuilles.
Par « phase de prolifération de cellules de *Tripterygium wilfordii* », on entend selon l'invention une phase dans laquelle les cellules de *Tripterygium wilfordii* sont en suspension dans un milieu de prolifération et dans des conditions adaptées à leur prolifération. Ces cellules pourront notamment être obtenues à partir de cals avant leur mise en suspension. Si nécessaire, les suspensions cellulaires pourront être régulièrement réensemencées afin de les maintenir dans des conditions de prolifération.
Par « cal » selon l'invention, on entend un amas de cellules dédifférenciées, également appelées cellules souches ou cellules méristématiques.
Par « environ », on entend dans la présente description que la valeur concernée peut être inférieure ou supérieure de 10%, notamment de 5%, en particulier de 1%, à la valeur indiquée. L'induction de cals pourra être obtenue par toute méthode connue de l'homme du métier. Les cals selon l'invention pourront notamment être obtenus de la manière décrite ci-après. L'induction de cals à partir d'un explant de tissu de partie de plante, notamment une partie aérienne, telle qu'une feuille, de *Tripterygium wilfordii,* est bien connue de l'homme du métier. L'induction de cals pourra notamment être réalisée par :

- obtention d'un explant de tissu de la plante, par exemple un morceau de feuille de taille de 1 $cm^2$ environ,
- mise en culture de l'explant sur un milieu de prolifération gélosé (par exemple par ajout de 4 à 12 g/L d'agar, par exemple environ 8 g/L d'agar, au milieu de prolifération selon l'invention)
- incubation, notamment à l'obscurité, à une température d'environ 25-30°C, par exemple à environ 27 à 28°C.

*Etape (i)* : *phase de prolifération de cellules*

L'homme du métier connaissant les cultures de cellules d'une plante comme *Tripterygium wilfordii* pourra facilement déterminer la composition du milieu de prolifération nécessaire à leur prolifération. Préférentiellement, ce milieu de prolifération permettra la prolifération des cellules sous formes dédifférenciées, c'est-à-dire sous formes totipotentes. Le maintien sous forme dédifférenciée pourra notamment être obtenu par l'utilisation de ratios particuliers cytokinines/auxines dans le milieu de prolifération. Le milieu de prolifération pourra notamment comprendre :

- Au moins un macroélément, notamment choisi parmi $NH_4NO_3$, $KNO_3$, $CaCl_2.2H_2O$, $MgSO_4.7H_2O$, $KH_2PO_4$ et un mélange de ceux-ci, par exemple à une concentration en macroéléments totale comprise entre 1000 et 9000 mg/L, par exemple entre 3000 et 8000 mg/L de milieu de prolifération : lors de la phase de prolifération, la concentration en macroéléments totale du milieu de prolifération sera notamment comprise entre 3000 et 5500 mg/L de milieu de prolifération ;
- Au moins un microélément, notamment choisi parmi KI, $H_3BO_3$, $MnSO_4.4H_2O$, $ZnSO_4.H_2O$, $Na_2MoO_4.2H_2O$, $CuSO_4.5H_2O$, $CoCl_2.6H_2O$, $FeSO_4.7H_2O$, $Na_2EDTA.2H_2O$ et un mélange de ceux-ci, par exemple à une concentration en microéléments totale comprise entre 10 et 200 mg/L, notamment entre 50 et 150 mg/L de milieu de prolifération ;
- Au moins une vitamine, notamment choisie parmi le myo-inositol, l'acide nicotinique, la pyridoxine-HCl, la thiamine-HCl et un mélange de ceux-ci, par exemple à une concentration en vitamines totale pouvant aller de 0,01 à 3g/L, notamment de 0,05 à 1g/L de milieu de prolifération ;
- Au moins un acide aminé, notamment la glycine, par exemple à une concentration en acides aminés totale pouvant aller de 0,15 à 5 mg/L, notamment entre 1 et 4mg/L de milieu de prolifération : lors de la phase de prolifération, la concentration en acides aminés du milieu de prolifération sera notamment comprise entre 1 et 2,5mg/L de milieu de prolifération ;
- Au moins une source de carbone, notamment du saccharose, par exemple à une concentration totale en source de carbone de 10 à 70 g/L de milieu de prolifération, par exemple à environ 30 g/L ;
- Au moins une hormone végétale (encore appelée hormone de croissance végétale ou facteur de croissance végétale ou régulateur de croissance végétale) notamment choisie parmi une ou plusieurs cytokinines, notamment la kinétine et/ou la 6-furfurylaminopurine, une ou plusieurs auxines, notamment l'acide 2,4-dichlorophénoxyacétique (2,4 D) et/ou l'acide naphtalène acétique (NAA), et un mélange de celles-ci. Lors de la phase de prolifération, le milieu de prolifération comprendra notamment au moins une cytokinine et au moins une auxine. Les hormones de croissance végétale seront notamment ajoutées au milieu de prolifération à une concentration et à un ratio permettant la prolifération des cellules sous forme dédifférenciées. Elles seront notamment choisies parmi la kinétine, la 6-furfurylaminopurine, l'acide 2,4 D, l'acide NAA et un mélange de ceux-ci ; notamment choisies parmi la kinétine, l'acide 2,4 D, l'acide NAA et un mélange de ceux-ci. Il pourra s'agir en particulier d'un mélange de kinétine, acide 2,4 D

et acide NAA.

La concentration en auxines sera notamment comprise entre 0,001 et 10mg/L de milieu de prolifération, par exemple entre 0,1 et 3mg/L de milieu de prolifération.
La concentration en cytokinines sera notamment comprise entre 0,01 et 0,5mg/L de milieu de prolifération, par exemple entre 0,05 et 0,15 mg/L de milieu de prolifération.
Dans un mode de réalisation, le ratio hormonal auxines / cytokinines sera compris entre 0,2 à 2,5 / 0,01 à 0,5, notamment entre 1 à 2 / 0,05 à 0,2, notamment sera d'environ 1,5 / 0,1.
Notamment le milieu de prolifération selon l'invention pourra comprendre 1,5 mg/L d'auxines, notamment d'acide 2,4 D et d'acide NAA, et 0,1 mg/L de cytokinines, notamment de kinétines.

Le milieu de prolifération sera avantageusement stérile et préférentiellement à un pH proche de la neutralité.

Un exemple de milieu de prolifération adapté à la prolifération des cellules d'une plante *Tripterygium wilfordii* selon l'invention est notamment décrit par Murashige & Skoog (Physiologia Plantarum, 1962, 15 : 473-497) ou selon l'exemple 2 de la présente demande.

Ce milieu de prolifération peut par exemple avoir la composition suivante (les concentrations sont exprimées par rapport au volume de milieu de prolifération sans cellules) : Macroéléments : $NH_4NO_3$ à 1650mg/L, $KNO_3$ à 1900 mg/L, $CaCl_2.2H_2O$ à 440 mg/L, $MgSO_4.7H_2O$ à 370 mg/L, $KH_2PO_4$ à 170 mg/L ; Microéléments : KI à 0,83 mg/L, $H_3BO_3$ à 6,2 mg/L, $MnSO_4.4H_2O$ à 22,3 mg/L, $ZnSO_4.H_2O$ à 6,6 mg/L, $Na_2MoO_4.2H_2O$ à 0,25 mg/L, $CuSO_4.5H_2O$ à 0,025 mg/L, $CoCl_2.6H_2O$ à 0,025 mg/L, $FeSO_4.7H_2O$ à 27,8 mg/L, $Na_2EDTA.2H_2O$ à 37,3 mg/L ; Vitamines : myo-inositol à 100 mg/L, acide nicotinique à 0,5 mg/L, pyridoxine-HCl à 0,5 mg/L, thiamine-HCl à 0,5 mg/L ; Acides aminés : glycine à 2 mg/L ; Source de carbone : saccharose à 30 g/L ; et Hormones végétales : acide NAA à 1 mg/L, acide 2,4 D à 0,5 mg/L, kinétine à 0,1mg/L, le tout ajusté à pH 6 avant stérilisation, par exemple par autoclavage de 20 minutes à 121°C ou par filtration sur filtre de 0,2$\mu$m.

Alternativement, le milieu de prolifération peut avoir la composition suivante (les concentrations sont exprimées par rapport au volume de milieu de prolifération sans cellules) : Macroéléments : $NH_4NO_3$ à 1650 mg/L, $KNO_3$ à 2500 mg/L, $CaCl_2.2H_2O$ à 440 mg/L, $MgSO_4.7H_2O$ à 370 mg/L, $KH_2PO_4$ à 130 mg/L ; Microéléments : KI à 0,41 mg/L, $H_3BO_3$ à 6,2 mg/L, $MnSO_4.4H_2O$ à 22,3 mg/L, $ZnSO_4.H_2O$ à 7,5 mg/L, $Na_2MoO_4.2H_2O$ à 0,25 mg/L, $CuSO_4.5H_2O$ à 0,025 mg/L, $CoCl_2.6H_2O$ à 0,025 mg/L, $FeSO_4.7H_2O$ à 19,85 mg/L, $Na_2EDTA.2H_2O$ à 26,64 mg/L ; Vitamines : myo-inositol à 50 mg/L, acide nicotinique à 0,25 mg/L, pyridoxine-HCl à 0,25 mg/L, thiamine-HCl à 0,25 mg/L ; Source de carbone : saccharose à 30 g/L ; et Hormones végétales : acide NAA à 0,35 mg/L, acide 2,4 D à 0,575 mg/L, kinétine à 0,083 mg/L.

Alternativement, le milieu de prolifération peut avoir la composition suivante (les concentrations sont exprimées par rapport au volume de milieu de prolifération sans cellules) : Macroéléments : $NH_4NO_3$ à 20 mM, $KNO_3$ à 19 mM, $CaCl_2.2H_2O$ à 3mM, $MgSO_4.7H_2O$ à 1,5 mM, $KH_2PO_4$ à 1,2 mM, KI à 0,005 mM, $H_3BO_3$ à 0,1 mM, $MnSO_4.4H_2O$ à 0,1 mM, $ZnSO_4.H_2O$ à 0,04 mM, $Na_2MoO_4.2H_2O$ à 0,001 mM, $CuSO_4.5H_2O$ à 0,0001 mM, $CoCl_2.6H_2O$ à 0,0001 mM, $FeSO_4.7H_2O$ à 0,1 mM, $Na_2EDTA.2H_2O$ à 0,1 mM, myo-inositol à 0,5 mM, acide nicotinique à 0,004 mM, pyridoxine-HCl à 0,002 mM, thiamine-HCl à 0,0015 mM, glycine à 0,03 mM, saccharose à 87,6 mM, acide NAA à 0,005 mM, acide 2,4 D à 0,002 mM, kinétine à 0,0005 mM.

L'ensemencement du milieu de prolifération pourra être réalisé à partir de la mise en suspension de cellules de cals à une concentration comprise entre 20 et 300 g dans 1L de milieu de prolifération et préférentiellement entre 100 et 200 g dans 1L de milieu de prolifération, par exemple environ 150 g dans 1L de milieu de prolifération.

La phase de prolifération aura lieu dans des conditions de multiplication de la biomasse.

Par « conditions de multiplication de la biomasse » selon l'invention, on entend notamment les conditions de température, de durée, d'agitation et de luminosité nécessaires à la prolifération des cellules en suspension. L'homme du métier connaissant les cultures de cellules de *Tripterygium wilfordii* pourra facilement déterminer les conditions de multiplication de la biomasse. Dans un mode de réalisation selon l'invention, l'étape de prolifération de la biomasse se fera à l'obscurité, à une température comprise entre 20 et 35°C, notamment entre 27 et 28°C, notamment à environ 27 ou 28°C, en particulier sous une agitation comprise entre 100 et 200 rpm, notamment à environ 125 rpm (orbitale de 22,5 mm) et pendant une durée comprise entre 10 et 30 jours, notamment 15 jours de culture.

Au cours de cette étape, les cellules peuvent être « repiquées » ou propagées, par exemple tous les 7 à 15 jours. Le repiquage des cellules est bien connu de l'homme du métier, il pourra notamment consister à diluer une partie de la culture cellulaire dans du milieu neuf concentré. Par exemple, 1/5$^{ème}$ de la culture est remise en suspension dans un volume de milieu neuf correspondant au volume de la culture initiale. Il permet l'entretien de la lignée cellulaire en milieu liquide dans un état de prolifération.

*Etape (ii) : phase d'élicitation*

Après la phase de prolifération, les cellules contenues dans la phase (i) sont élicitées par ajout d'un cocktail

d'élicitation. Le milieu de prolifération auquel a été ajouté le cocktail d'élicitation sera nommé « milieu d'élicitation ». La phase d'élicitation est la phase dans laquelle les cellules sont maintenues dans un état physiologique favorisant la biosynthèse de métabolites secondaires tels que les triterpènes pentacycliques.

La production de triterpènes pentacycliques a lieu, au cours de la phase d'élicitation, dans le cytosol de la cellule et peut diffuser en partie dans le milieu d'élicitation. La phase d'élicitation au sens de la présente invention correspond donc à la phase de production (biosynthèse) des triterpènes pentacycliques et plus particulièrement du Célastrol, de la Tingénine A et de la Tingénine B.

L'élicitation se fera préférentiellement après une phase de prolifération de 7 à 21 jours, notamment 12 à 20 jours, notamment 15, 16 ou 17 jours (notamment sans repiquage). Alternativement, l'ajout du cocktail d'élicitation pourra se faire lorsque la concentration en cellules obtenue lors de la phase de prolifération est double, notamment supérieure au double, par rapport à la concentration initiale en cellules dans le milieu de prolifération. L'ajout du cocktail d'élicitation pourra notamment se faire lorsque la concentration en cellules est supérieure à 200 g/L, par exemple entre 200 et 400 g/L, notamment environ 300 g/L (en quantité de cellules par litre de milieu de prolifération). Dans un mode de réalisation, l'ajout du cocktail d'élicitation se fera dans une culture dont la concentration en cellules est passée de 150 à 200 g/L lors du début de la phase de prolifération à une concentration comprise entre 300 et 400 g/L lors de la fin de la phase de prolifération.

A la suite de la phase de prolifération, les cellules végétales ont consommé la plupart, voire tous les éléments contenus dans le milieu de prolifération, et en particulier les sources de carbone telles que le saccharose. Il peut donc être nécessaire de rétablir la composition du milieu avant ou en même temps que l'ajout du cocktail d'élicitation.

Pour rétablir la composition du milieu, on peut notamment concentrer la culture cellulaire obtenue après l'étape de prolifération, par exemple par décantation ou filtration puis ajouter du milieu de prolifération neuf aux cellules ainsi obtenues. Dans ce cas, les cellules seront re-suspendues dans le milieu de prolifération neuf de manière à obtenir une concentration comprise entre 200 et 400 g/L, par exemple entre 250 et 350 g/L, notamment d'environ 300 g/L (en quantité de cellules par litre de milieu de prolifération).

Alternativement, on peut ajouter dans la culture cellulaire un mélange concentré permettant de rétablir les concentrations des éléments du milieu de prolifération. Le mélange concentré sera notamment ajouté juste avant, juste après ou en même temps que le cocktail d'élicitation. Par exemple on peut remplacer 1/5$^{ème}$ de la culture cellulaire par un volume équivalent du milieu de prolifération concentré 5 fois.

On considère que la concentration en éléments du milieu de prolifération est proche ou équivalente à zéro après 14, 15 ou 16 jours de phase de prolifération. En particulier, on considère que la concentration en éléments minéraux (plus particulièrement les macroéléments et les microéléments) et carbonés (plus particulièrement les sources de carbone) est proche de ou équivalente à zéro après 14, 15 ou 16 jours de phase de prolifération.

Dans un mode de réalisation, le milieu de prolifération selon l'invention au début de la phase d'élicitation comprendra entre autres :

- Au moins un macroélément, notamment choisi parmi $NH_4NO_3$, $KNO_3$, $CaCl_2.2H_2O$, $MgSO_4.7H_2O$, $KH_2PO_4$ et un mélange de ceux-ci, par exemple à une concentration en macroéléments totale comprise entre 5000 et 8000 mg/L, préférentiellement supérieure à 6000 mg/L de milieu de prolifération, avantageusement entre 6000 et 8000 mg/L ;
- Au moins un microélément, notamment choisi parmi KI, $H_3BO_3$, $MnSO_4.4H_2O$, $ZnSO_4.H_2O$, $Na_2MoO_4.2H_2O$, $CuSO_4.5H_2O$, $CoCl_2.6H_2O$, $FeSO_4.7H_2O$, $Na_2EDTA.2H_2O$ et un mélange de ceux-ci, par exemple à une concentration en microéléments totale comprise entre 10 et 200 mg/L, notamment entre 50 et 150 mg/L de milieu de prolifération ;
- Au moins une vitamine, notamment choisie parmi le myo-inositol, l'acide nicotinique, la pyridoxine-HCl, la thiamine-HCl et un mélange de ceux-ci, par exemple à une concentration en vitamines totale pouvant aller de 0,01 à 3g/L, notamment de 0,05 à 1g/L de milieu de prolifération ;
- Au moins un acide aminé, notamment la glycine, par exemple à une concentration dans le milieu de prolifération comprise entre 3 et 4 mg/L de milieu de prolifération ;
- Au moins une source de carbone, notamment du saccharose, par exemple à une concentration totale en source de carbone de 10 à 70 g/L de milieu de prolifération, par exemple à environ 30 g/L.

Dans un mode de réalisation le milieu de prolifération au début de la phase d'élicitation ne comprendra pas, ou comprendra une quantité négligeable, notamment moins de 0,001 g/mL, de cytokinine et d'auxine.

Le milieu de prolifération lors de la phase d'élicitation sera avantageusement stérile et préférentiellement à un pH proche de la neutralité.

Le milieu de prolifération lors de la phase d'élicitation pourra notamment avoir la composition suivante : Macroéléments : $NH_4NO_3$ à 2,8 g/L, $KNO_3$ à 3 g/L, $CaCl_2.2H_2O$ à 0,45 g/L, $MgSO_4.7H_2O$ à 74 mg/L, $KH_2PO_4$ à 34 mg/L ; Microéléments : KI à 0,16 mg/L, $H_3BO_3$ à 6,2 mg/L, $MnSO_4.4H_2O$ à 18,5 mg/L, $ZnSO_4.H_2O$ à 6,6 mg/L, $Na_2MoO_4.2H_2O$ à 0,25 mg/L, $CuSO_4.5H_2O$ à 0,025 mg/L, $CoCl_2.6H_2O$ à 0,025 mg/L, $FeSO_4.7H_2O$ à 28 mg/L, $Na_2EDTA.2H_2O$ à 37 mg/L ; Vitamines : myo-inositol à 250 mg/L, acide nicotinique à 1,7 mg/L, pyridoxine-HCl à 1

mg/L, thiamine-HCl à 1 mg/L ; Acides aminés : glycine à 4 mg/L ; Source de carbone : saccharose à 30 g/L.

Par « cocktail d'élicitation » selon l'invention, on entend un cocktail permettant de stopper la division cellulaire. Ce cocktail d'élicitation comprend au moins un éliciteur de type composé monocarboxylique et au moins un éliciteur biotique. Le cocktail d'élicitation est introduit, par exemple, à l'aide de solutions mères concentrées dans le milieu de culture.

Par « éliciteur de type composé monocarboxylique » selon l'invention, on entend plus particulièrement un éliciteur choisi dans le groupe comprenant, de préférence constitué par, l'acide 5-chloro salicylique, l'acide salicylique, l'acide acétylsalicylique, un ester de méthyle, notamment le jasmonate de méthyle, et un mélange de ceux-ci. Dans un mode de réalisation selon l'invention, l'éliciteur de type composé monocarboxylique est le jasmonate de méthyle, l'acide salicylique et/ou l'acide 5-chloro salicylique, notamment le jasmonate de méthyle. L'éliciteur de type composé monocarboxylique sera notamment ajouté de façon à obtenir une concentration finale comprise entre 0,005 et 0,1 g/L, notamment 0,01 et 0,05 g/L de milieu d'élicitation, notamment de 0,002 et 0,004 g/L de milieu d'élicitation.

Par « éliciteur biotique » selon l'invention, on entend plus particulièrement un éliciteur biotique choisi dans le groupe comprenant, de préférence constitué par, une N-acétylaminoglucosamine, notamment la chitine, le chitosan, les extraits de microorganismes ou de champignons, les oligosaccharides (polysaccharides, pectines, cellulose). Dans un mode de réalisation, l'éliciteur biotique est la chitine. La chitine est un polymère linéaire de motif répétitif : béta-1,4 N-acétyl D-glucosamine. L'éliciteur biotique sera notamment ajouté de façon à obtenir une concentration finale comprise entre 0,05 et 50 g/L de milieu d'élicitation, par exemple de 0,1 à 10 g/L, par exemple de 0,5 à 7 g/L, notamment de 1 à 5 g/L de milieu d'élicitation.

Dans un mode de réalisation, le cocktail d'élicitation comprend du jasmonate de méthyle, notamment à une concentration finale dans le milieu d'élicitation entre 0,002 et 0,005 g/L, et de la chitine, notamment à une concentration finale entre 1 et 4 g/L de milieu d'élicitation.

Dans un mode de réalisation, le cocktail d'élicitation selon l'invention comprendra en outre, au moins un facteur de différenciation cellulaire des cellules végétales et/ou au moins un précurseur de la voie de synthèse des terpènes.

Le « facteur de différenciation cellulaire des cellules végétales » selon l'invention pourra notamment être choisi dans le groupe comprenant, de préférence constitué par, une cytokinine, notamment la benzyl-aminopurine, l'acide abscissique, la kinétine, le thidiazuron, la 6-γ,γ-diméthylallylaminopurine (ou isopenténypladénine) ou la zéatine, une gibbérelline et un mélange de celles-ci, notamment la benzyl-aminopurine et/ou la 6-γ,γ-diméthylallylaminopurine, en particulier la 6-γ,γ-diméthylallylaminopurine.

Le « précurseur de synthèse des terpènes » selon l'invention pourra notamment être choisi dans le groupe comprenant, de préférence constitué par : le pyruvate de sodium, le pyrophosphate de potassium, l'acide mévalonique, le géraniol, le farnésol, l'isopentényle, le diméthylallyle, y compris leurs formes pyrophosphatées, l'acétate de sodium, l'acide pyruvique et leurs mélanges, notamment le géraniol, le farnésol, le pyruvate de sodium, le pyrophosphate de potassium et leurs mélanges, tel que le pyruvate de sodium et/ou le pyrophosphate de potassium. La benzyl-aminopurine (BAP) pourra notamment être utilisée à une concentration finale dans le milieu d'élicitation comprise entre 0,01 et 5 mg/L, par exemple entre 0,5 et 5 mg/L de milieu d'élicitation.

L'acide 5-chloro salicylique (5-Chloro SA) pourra notamment être utilisé à une concentration finale dans le milieu d'élicitation comprise entre 0,1 et 15 mg/L.

L'acide salicylique pourra notamment être utilisé à une concentration finale dans le milieu d'élicitation comprise entre 0,1 et 100 mg/L, par exemple entre 20 et 60 mg/L, par exemple environ 45 mg/L.

Le farnésol pourra notamment être utilisé à une concentration finale dans le milieu d'élicitation comprise entre 1 à 100 mg/L, par exemple entre 15 et 30 mg/L, par exemple à environ 30 mg/L. Le géraniol pourra notamment être utilisé à une concentration finale dans le milieu d'élicitation comprise entre 1 et 100 mg/L, par exemple entre 20 et 30 mg/L.

Le pyruvate de sodium pourra notamment être utilisé à une concentration finale dans le milieu d'élicitation comprise entre 100 et 5000 mg/L, par exemple entre 500 et 2000 mg/L.

Le pyrophosphate de potassium pourra notamment être utilisé à une concentration finale dans le milieu d'élicitation comprise entre 1 et 2000 mg/L, par exemple entre 100 et 1000 mg/L de milieu d'élicitation.

La 6-γ,γ-diméthylallylaminopurine (encore appelée 2iP ou isopentényladénine) pourra notamment être utilisée à une concentration finale dans le milieu d'élicitation comprise entre 0,005 et 10 mg/L, par exemple entre 0,01 et 3 mg/L, par exemple entre 0,1 et 2 mg/L de milieu d'élicitation.

Dans un mode de réalisation de l'invention, le cocktail d'élicitation comprend du jasmonate de méthyle, de la chitine, du pyruvate de sodium, du pyrophosphate de potassium ou un mélange de ceux-ci.

Dans un mode de réalisation de l'invention, le cocktail d'élicitation comprend du jasmonate de méthyle, de la chitine, du pyruvate de sodium, du pyrophosphate de potassium et éventuellement de la benzyl-aminopurine et/ou de la 6-γ,γ-diméthylallylaminopurine.

Dans un mode de réalisation de l'invention, le cocktail d'élicitation comprend ou consiste en (les concentrations données entre parenthèses correspondent à la concentration dans le milieu d'élicitation, le cocktail initial pouvant être plus ou moins concentré en fonction de la dilution envisagée) du pyruvate de sodium (de 500 à 2000 mg/L), du pyrophosphate de potassium (de 100 à 1000 mg/L), de la 6-γ,γ-diméthylallylaminopurine (de 0,1 à 2 mg/L), du jasmonate de méthyle (de

0,002 à 0,005 g/L) et de la chitine (de 1 à 4 g/L).

Dans un mode de réalisation de l'invention, le cocktail d'élicitation comprend ou consiste en (les concentrations données entre parenthèses correspondent à la concentration dans le milieu d'élicitation, le cocktail initial pouvant être plus ou moins concentré en fonction de la dilution envisagée) de la benzyl-aminopurine (de 0,5 à 5 mg/L, notamment de 0,5 à 3 mg/L), de l'acide 5-chloro salicylique (de 2 à 6 mg/L, notamment de 3 à 5 mg/L, par exemple à environ 3 ou à environ 5 mg/L), de l'acide acétylsalicylique et/ou de l'acide salicylique (de 20 à 60 mg/L, notamment de 30 à 50 mg/L, notamment de 33 à 45 mg/L), du jasmonate de méthyle (de 0,002 à 0,05 g/L, notamment de 10 à 40 mg/L), de la chitine (de 1 à 4 g/L), et du farnésol (de 19 à 40 mg/L) et/ou du géraniol (de 20 à 30 mg/L).

Dans un mode de réalisation de l'invention, le cocktail d'élicitation comprend ou consiste en (les concentrations données entre parenthèses correspondent à la concentration dans le milieu d'élicitation, le cocktail initial pouvant être plus ou moins concentré en fonction de la dilution envisagée) du pyruvate de sodium (de 500 à 2000 mg/L), du pyrophosphate de potassium (de 100 à 1000 mg/L), de la 6-$\gamma$,$\gamma$-diméthylallylaminopurine (de 0,1 à 1 mg/L), du jasmonate de méthyle (de 0,002 à 0,05 g/L, notamment de 10 à 40 mg/L) et de la chitine (de 1 à 4 g/L).

Dans un mode de réalisation de l'invention, le cocktail d'élicitation comprend ou consiste en (les concentrations données entre parenthèses correspondent à la concentration dans le milieu d'élicitation, le cocktail initial pouvant être plus ou moins concentré en fonction de la dilution envisagée) du pyruvate de sodium (environ 1,5 g/L), du pyrophosphate de potassium (environ 0,4 g/L), de la 6-$\gamma$,$\gamma$-diméthylallylaminopurine (environ 0,4 mg/L), du jasmonate de méthyle (environ 0,03 g/L), et de la chitine (environ 2 g/L).

Pendant la phase d'élicitation, après ajout du cocktail d'élicitation, la culture est maintenue sous agitation, notamment entre 50 et 200 rpm, notamment à environ 125 rpm, pendant une période comprise entre 3 et 30 jours, notamment entre 10 et 25 jours, notamment de 12 à 15 jours, en particulier à une température comprise entre 20 et 35°C, notamment à environ 27°C et avantageusement avec un taux d'oxygène dissout dans le milieu de culture de 2 à 40%, préférentiellement à environ 16%. Un apport en air stérile suffisamment enrichi en oxygène pourra être fourni si nécessaire notamment dans le volume mort du bioréacteur ou par diffusion dans le milieu. De préférence, la phase d'élicitation (ii) est menée à l'obscurité. Lors de la phase d'élicitation, la culture cellulaire n'est préférentiellement pas repiquée.

*Etape (iii) : phase de préparation de l'extrait comprenant, notamment enrichi en, triterpènes pentacycliques*

Après la phase d'élicitation, le procédé comprend une étape de préparation d'un extrait comprenant, notamment enrichi en, triterpènes pentacycliques.

L'extrait pourra notamment être obtenu par séparation de la biomasse et du surnageant de culture. La séparation pourra notamment être faite par filtration directe (0-50 $\mu$m), par centrifugation ou par décantation des cellules.

Dans un mode de réalisation, l'extrait pour une utilisation selon l'invention pourra consister en le surnageant de culture ainsi récupéré.

L'extrait pourra également être obtenu après la lyse de la biomasse. Par exemple, les cellules contenues dans la biomasse récupérée pourront être lysées par une méthode physique (sonication ou broyage) ou chimique (lyse acide) puis ce lysat sera soumis à une extraction par solvant (appelé solvant d'extraction). La phase organique, comprenant notamment les triterpènes issus du cytosol, est ensuite récupérée, notamment par décantation ou centrifugation. Le solvant sera notamment un solvant de type ester, et plus particulièrement un acétate d'alkyle, l'alkyle étant plus particulièrement linéaire ou ramifié à 1 à 6 atomes de carbone, notamment l'acétate d'éthyle ou l'acétate d'isopropyle. Le volume de solvant utilisé sera notamment de 2 volumes par poids de biomasse.

Préférentiellement, l'extrait pour une utilisation selon l'invention correspondra à la phase organique ainsi récupérée, éventuellement concentrée partiellement ou totalement, c'est-à-dire que le solvant d'extraction est évaporé partiellement ou totalement.

Alternativement, l'extrait pourra être obtenu après évaporation du solvant et éventuellement sa substitution notamment par un support adapté au domaine d'utilisation de l'extrait (cosmétique, pharmaceutique) et notamment par des huiles végétales ou des solvants tel que notamment le pentylène glycol (ou pentiol), ou du Myritol 318®.

L'extrait ainsi obtenu pourra éventuellement être purifié de sorte à obtenir un extrait purifié d'un ou plusieurs triterpènes pentacycliques. L'extrait selon l'invention, et plus particulièrement l'extrait purifié, comprendra avantageusement 90% ou plus, notamment 95% ou plus, notamment 98% ou plus en poids d'un ou plusieurs triterpènes pentacycliques selon l'invention par rapport au poids total de l'extrait sec.

Dans un mode de réalisation, l'extrait selon l'invention, notamment l'extrait enrichi ou purifié, comprend 60% ou plus, notamment 80% ou plus, notamment 85% ou plus, notamment 90% ou plus, notamment 95% ou plus, notamment plus 98% ou plus, notamment 100% de Célastrol en poids par rapport au poids total de l'extrait. Il pourra comprendre notamment entre 50 et 98%, notamment entre 70 et 90%, notamment entre 76 et 84% de Célastrol en poids par rapport au poids total de l'extrait sec.

Dans un mode de réalisation, l'extrait selon l'invention, notamment l'extrait enrichi ou purifié, comprend entre 1 et 20%, notamment entre 5 et 15%, notamment entre 8 et 12% de Tingénine A en poids par rapport au poids total de l'extrait

sec.

Dans un mode de réalisation, l'extrait selon l'invention, notamment l'extrait enrichi ou purifié, comprend entre 1 et 20%, notamment entre 5 et 15%, notamment entre 8 et 12% de Tingénine B en poids par rapport au poids total de l'extrait sec.

Dans un mode de réalisation, l'extrait selon l'invention, notamment l'extrait enrichi ou purifié, comprend :

- Entre 50 et 98%, notamment entre 70 et 90%, notamment entre 76 et 84% de Célastrol en poids par rapport au poids total de l'extrait sec;
- Entre 1 et 20%, notamment entre 5 et 15%, notamment entre 8 et 12% de Tingénine A en poids par rapport au poids total de l'extrait sec ;
- Entre 1 et 20%, notamment entre 5 et 15%, notamment entre 8 et 12% de Tingénine B en poids par rapport au poids total de l'extrait sec.

La purification de l'extrait selon l'invention pourra notamment être réalisée par une phase de séparation du ou des triterpènes(s) pentacycliques(s), notamment le Célastrol, la Tingénine A et/ou la Tingénine B, notamment par fractionnement HPLC (Chromatographie liquide à haute performance), au cours duquel les pics à 426nm comprenant le Célastrol, la Tingénine A et la Tingénine B sortent respectivement à 19,75 min, 18,03 min et 16,1 min.

Selon un deuxième aspect, la présente invention concerne aussi une composition cosmétique ou dermatologique comprenant une association selon l'invention comprenant un extrait de myrte et un extrait de Tripterygium wilfordii, avec au moins un excipient cosmétiquement ou dermatologiquement acceptable.

Dans un mode de réalisation particulier, l'association selon l'invention est le seul principe actif de la composition cosmétique ou dermatologique selon l'invention.

L'invention vise de préférence des compositions cosmétique ou dermatologique selon l'invention se présentant sous une forme adaptée à une application topique.

Les compositions cosmétique ou dermatologique selon l'invention peuvent se présenter ainsi sous les formes qui sont habituellement connues pour une administration topique, c'est-à-dire notamment les lotions, les shampoings, les baumes, les mousses, les gels, les dispersions, les émulsions, les sprays, les sérums, les masques, les crèmes ou les sticks avec des excipients permettant notamment, pour certains, une pénétration afin d'améliorer les propriétés et l'accessibilité des principes actifs.

La composition cosmétique ou dermatologique selon l'invention comprendra un extrait de myrte qui est une fraction apolaire comprenant des myrtucommulones et l'acide ursolique. En particulier, les myrtucommulones comprennent les myrtucommulones A, B', D, B, l'isosemimyrtucommulone et la semimyrtucommulone.

La composition cosmétique ou dermatologique selon l'invention comprendra un extrait de *Tripterygium wilfordii* qui comprend au moins un triterpène pentacyclique, choisi parmi la Tingénine A, la Tingénine B et le Célastrol et leurs mélanges, tel que défini ci-dessus, et en particulier choisi parmi la Pristimérine, la Tripterygone et leurs mélanges, et en particulier un mélange de Tingénine A, Tingénine B et Célastrol. L'extrait de *Tripterygium wilfordii* sera un extrait de *Tripterygium wilfordii* comprenant, notamment enrichi en, triterpène(s) pentacyclique(s), choisi parmi la Tingénine A, la Tingénine B et/ou le Célastrol, et plus particulièrement un extrait de *Tripterygium wilfordii* comprenant, notamment enrichi en, triterpène(s) pentacyclique(s) susceptible d'être obtenu par le procédé décrit ci-dessus et qui comprend les étapes suivantes :

(i) Une phase de prolifération de cellules de *Tripterygium wilfordii* dans un milieu de prolifération,
(ii) Une phase d'élicitation par ajout d'un cocktail d'élicitation à la culture cellulaire obtenue à l'étape (i), ledit cocktail d'élicitation comprenant au moins un éliciteur de type composé monocarboxylique et au moins un éliciteur biotique, et
(iii) La préparation d'un extrait comprenant, notamment enrichi en, triterpènes pentacycliques à partir de la culture cellulaire obtenue à l'étape (ii).

La composition cosmétique ou dermatologique selon l'invention comprendra notamment entre 0,01 et 1% d'un extrait de *Tripterygium wilfordii* en poids d'extrait sec par rapport au poids de la composition totale, notamment entre 0,05 et 0,8%, notamment entre 0,1 et 0,5%, notamment entre 0,2 et 0,4% en poids d'extrait sec par rapport au poids de la composition totale. Selon un mode préféré de réalisation, la composition cosmétique ou dermatologique selon l'invention comprend environ 0,3% d'un extrait de *Tripterygium wilfordii* en poids d'extrait sec par rapport au poids de la composition totale.

L'extrait de *Tripterygium wilfordii* comprendra avantageusement 90% ou plus de triterpène(s) pentacyclique(s) en poids par rapport au poids total de l'extrait sec. Dans un mode de réalisation avantageux, l'extrait de *Tripterygium wilfordii* comprendra entre 1 et 20% de Tingénine A en poids par rapport au poids total de l'extrait sec, entre 1 et 20% de Tingénine B en poids par rapport au poids total de l'extrait et au moins 60% de Célastrol en poids par rapport au poids total de l'extrait sec.

La composition cosmétique ou dermatologique selon l'invention comprendra avantageusement entre 0,01 à 1% d'un extrait de myrte en poids d'extrait sec par rapport au poids de la composition totale, notamment entre 0,05 et 0,8%, notamment entre 0,08 et 0,4%, notamment entre 0,08 et 0,2% en poids d'extrait sec par rapport au poids de la composition totale. Selon un mode préféré de réalisation, la composition cosmétique ou dermatologique selon l'invention comprend environ 0,1% d'un extrait de myrte en poids d'extrait sec par rapport au poids de la composition totale.

L'extrait de myrte comprendra avantageusement entre 3 et 10% de myrtucommulones en poids par rapport au poids total de l'extrait sec et/ou entre 10 et 30% d'acide ursolique en poids par rapport au poids total de l'extrait sec.

Selon un troisième aspect, l'invention concerne une association selon l'invention comprenant un extrait de myrte et un extrait de *Tripterygium wilfordii* ou une composition cosmétique ou dermatologique comprenant une telle association avec au moins un excipient cosmétiquement ou dermatologiquement acceptable, pour son utilisation dans le traitement de l'inflammation induite par *C. acnes.*

L'invention concerne également une association selon l'invention comprenant un extrait de myrte et un extrait de *Tripterygium wilfordii* ou une composition cosmétique ou dermatologique comprenant une telle association avec au moins un excipient cosmétiquement ou dermatologiquement acceptable, pour son utilisation dans le traitement de l'acné ou des peaux acnéiques.

Les exemples qui suivent illustrent l'invention sans en limiter la portée.

## DESCRIPTION DES FIGURES

La figure 1 représente le chromatogramme de l'HPLC obtenu pour l'extrait « CCV » de *Tripterygium wilfordii* selon l'exemple 2 contenant les triterpènes pentacycliques suivants : Célastrol, Tingénine A et Tingénine B.

## EXEMPLES

### Exemple 1 : Préparation d'un extrait de myrte

1 kg de feuilles de myrte broyées sont extraites par 5 volumes d'acétate d'isopropyle sous agitation à reflux pendant 1 heure. Après filtration et rinçage du marc, les jus d'extraction sont décolorés par addition de charbon actif. Après filtration, le filtrat décoloré est concentré jusqu'à 2 litres puis séché sur éthanol jusqu'à l'élimination de l'acétate d'isopropyle. La phase aqueuse obtenue est ensuite désodorisée par traitement thermique, puis séchée par lyophylisation. 1 kg de feuilles de myrte permet d'obtenir 25 g environ d'extrait sec de myrte. Celui-ci renferme 7% de myrtucommulones et 25% d'acide ursolique.

### Exemple 2 : Obtention d'un extrait de culture de cellules végétales (CCV) de *Tripterygium wilfordii* enrichi en triterpènes pentacycliques

On réalise une culture dans un réacteur de type Wave (volume 5 L) de Sartorius Stedim Biotech (Allemagne). On inocule le réacteur avec une suspension de cellules de *Tripterygium wilfordii* issus d'un Erlenmeyer. Le milieu de prolifération a, par exemple, la composition indiquée ci-dessous :

Macroéléments : $NH_4NO_3$ à 1650 mg/L, $KNO_3$ à 2500 mg/L, $CaCl_2.2H_2O$ à 440 mg/L, $MgSO_4.7H_2O$ à 370 mg/L, $KH_2PO_4$ à 130 mg/L ;
Microéléments : KI à 0,41 mg/L, $H_3BO_3$ à 6,2 mg/L, $MnSO_4.4H_2O$ à 22,3 mg/L, $ZnSO_4.H_2O$ à 7,5 mg/L, $Na_2MoO_4.2H_2O$ à 0,25 mg/L, $CuSO_4.5H_2O$ à 0,025 mg/L, $CoCl_2.6H_2O$ à 0,025 mg/L, $FeSO_4.7H_2O$ à 19,85 mg/L, $Na_2EDTA.2H_2O$ à 26,64 mg/L ;
Vitamines : myo-inositol à 50 mg/L, acide nicotinique à 0,25 mg/L, pyridoxine-HCl à 0,25 mg/L, thiamine-HCl à 0,25 mg/L ;
Source de carbone : saccharose à 30 g/L ;
Hormones végétales : acide NAA à 0,35 mg/L, acide 2,4 D à 0,575 mg/L, kinétine à 0,083 mg/L.

Le pH du milieu est ajusté à pH 6 $\pm$ 0,5 (par ajout de KOH, 1M) avant un traitement de stérilisation approprié, par exemple autoclave à 121°C pour une durée minimale de 20 minutes ou par filtration stérilisante sur 0,2 $\mu$m.

Sous agitation en continu, après avoir atteint le maximum en biomasse environ 17 jours après, on procède à l'élicitation. Le cocktail d'élicitation est ensuite ajouté dans l'Erlenmeyer au milieu de prolifération à l'aide de solutions mères réalisées dans du diméthylsulfoxyde. La composition du cocktail éliciteur permet d'obtenir les concentrations suivantes dans le milieu d'élicitation (+ cellules) : pyruvate de sodium 1,5 g/L, pyrophosphate de potassium 0,44 g/L, 2iP 0,0004 g/L, jasmonate de méthyle 0,036 g/L et chitine 2 g/L. On arrête la culture après 15 jours d'élicitation. La majorité de la biomasse est récupérée par filtration de la suspension cellulaire avec un filtre nylon (20-50$\mu$m). A partir de 5 L de

suspension, on récupère environ 1925 g de biomasse. Cette biomasse est extraite avec de l'acétate d'éthyle (ou encore de l'acétate d'isopropyle) en proportion 2 : 1 (Volume : Poids) par rapport au poids de biomasse (ici 3850 mL de solvant pour 1925 g de biomasse). Le mélange biomasse/solvant est alors soumis à une extraction physique, par sonication ou par broyage. La phase organique est alors récupérée après macération sous agitation. L'ajout du solvant (suivi d'une macération sous agitation et de la récupération de la phase organique) est répété deux fois. On a concentré le solvant sous vide, puis solubilisé le concentrat dans du pentylène glycol, et mis le mélange sous vide afin d'éliminer le solvant organique résiduel. On obtient alors une solution nommée « extrait CCV » contenant les triterpènes pentacycliques suivants : Célastrol, Tingénine A et Tingénine B, comme déterminé par le chromatogramme de l'HPLC présenté en Figure 1. Conditions chromatographie HPLC : appareil de chromatographie liquide Alliance (Waters 2695 version 2.03) ; Colonne Sunfire C18, 100Å, 5 $\mu$m (4.6 mm X 150 mm) ; gradient de solvants eau/acétonitrile, débit 3 ml/min ; détection des triterpènes à $\lambda$ 460 nm.

**Exemple 3 : Propriétés biologiques de l'association selon l'invention**

Le but de cette étude est d'évaluer les propriétés anti-inflammatoires d'un extrait de myrte et d'un extrait de *Triptérygium wilfordii* sur l'inflammation induite par *C. acnes* organisé en biofilm ou par un extrait membranaire de *C. acnes.* Pour adresser cette hypothèse, des concentrations sous-optimales d'extrait de myrte, d'extrait de *Triptérygium wilfordii* et de leur association sont évaluées sur la cascade immuno-inflammatoire induite en réponse au phylotype pro-pathogène de C. *acnes* (phylotype IA1), en amont de la voie Th17, sur la production de cytokines pro-inflammatoires par des monocytes.

Méthode :

Les expériences sont menées sur des cocultures de cellules dendritiques immatures dérivées de monocytes, à la concentration finale de $1.10^5$ cellules/ml/puit et de biofilms ou membranes de *C. acnes* de phylotype IA1. Les monocytes sont purifiés à partir de sang humain par sélection négative (The EasySep™ Human Monocyte Enrichment Kit, StemCell).
Au Jour 0, les monocytes sont incubés dans un milieu de différenciation (Gibco Roswell Park Memorial Institute (RPMI), 10% v/v Sérum de Veau Fœtal (SVF) décomplémenté (chauffé à 56°C pendant 30 min), 50ng/ml IL-4 et 100 ng/ml Recombinant Human Granulocyte Macrophage Colony-Stimulating Factor (GM-CSF)). Au 3$^{ème}$ jour de culture, la moitié du milieu est remplacé par du milieu frais. Au jour 6, les cellules dendritiques immatures sont caractérisées par cyrtométrie de flux et stimulées par un biofilm ou des membranes de *C. acnes.*

*Préparation des biofilms :*

Milieux utilisés :

- Gélose Columbia + 5% de sang de mouton (COS)
- Bouillon biofilm composé de $FeSO_4$ $7H_2O$ 0,005 g/L + $Na_2HPO_4$ 12,5 g/L + $KH_2PO_4$ 5g/L + vitamine Casamino acids 1g/L + Lactose 0,25 g/L + extrait de levure 1g/L

Préparation d'une suspension de *C. acnes* P52 directement dans le bouillon biofilm à une concentration de l'ordre de $10^8$ UFC/ml, par ajustement au spectromètre à environ 54% de transmission à 640nm.
Dénombrement de la suspension initiale par dilutions successives au 1/10$^{ème}$ jusqu'à la dilution $10^{-6}$ et étalement de 100$\mu$L des dilutions $10^{-5}$ à $10^{-6}$ sur gélose COS. Incubation pendant 72h sous anaérobiose à 36°C.
Pour l'obtention de biofilm, inoculation de l'ensemble des puits d'une microplaque de 24 puits avec 2mL de bouillon biofilm contenant une concentration de *C. acnes à* $10^8$ UFC/mL, incubation à 36°C sous anaérobiose. Après 24h d'incubation, renouvellement du milieu, les puits sont vidés, puis rincés doucement avec 2mL d'EDS (eau distillée stérile) (1 fois). 2mL de bouillon biofilm sont introduits dans chaque puits et les microplaques sont replacées en incubation à 36°C en anaérobiose. Après 48h d'incubation, le milieu est à nouveau renouvelé dans les mêmes conditions.
Les biofilms ainsi préparés sont lavés avec du milieu de culture (RPMI + 10% v/v SVF) avant incubation avec les cellules dendritiques.

*Membranes :*

Les membranes de *C. acnes* sont achetées au laboratoire Icare (Saint-Beauzire, France).
La stimulation des cellules dendritiques immatures dérivées des monocytes par les membranes de *C. acnes* est

réalisée avec une dilution au 1/20 d'une solution de membranes dans du milieu de culture (RPMI + 10% v/v SVF) à une concentration de 0,15 mg/ml.

La stimulation des cellules dendritiques dérivées des monocytes par les biofilms de *C. acnes* est réalisée au facteur de multiplicité d'infection (MOI) proche de 100 ;

$$MOI = [C.\ acnes] / [\text{cellules dendritiques dérivées des monocytes}].$$

L'extrait de myrte utilisé dans cette étude est préparé selon l'exemple 1. Cet extrait est solubilisé et stocké à 20 mg/ml dans de l'éthanol (10 mg d'extrait sec préparé selon l'exemple 1, dans 1 ml d'éthanol). La concentration testée est de 10μg/ml.

L'extrait de *Tripterygium wilfordii* (extrait Trip ci-dessous) utilisé dans cette étude est préparé selon l'exemple 2. Cet extrait est solubilisé et stocké à 10 mg/ml dans du milieu de culture (RPMI + 10% v/v SVF). La concentration testée est de 25 μg/ml.

Un contrôle positif pour inhiber la production des cytokines inflammatoires est également testé dans cette étude, il s'agit de la Dexamethasone à la concentration de 300 ng/ml, solubilisée dans de l'eau.

Les surnageants sont récupérés après 24 heures de stimulation.

Les résultats sont moyennés à partir de 3 expériences indépendantes.

Plusieurs cytokines sont évaluées dans cette étude : IL-6, IL-8, IL-10, et IL-12p40, ces cytokines sont quantifiées en multiplex via la technologie Luminex (Bioplex 200, Biorad).

Résultats :

*Stimulation par les membranes de C. acnes*

Les résultats sur l'inhibition de la production d'interleukines 6 (IL-6) par des cellules dendritiques dérivées des monocytes, stimulées avec les membranes de C. *acnes* sont présentés dans le tableau 1 ci-dessous.

[Tableau 1]

| Produits | Concentration | Inhibition (%) | Statistiques |
|---|---|---|---|
| Contrôle (Mb *C. acnes*) | - | 0 | - |
| Dexamethasone | 300 ng/ml | 76,8 | P<0,01 |
| Extrait de myrte | 10 μg/ml | 42,3 | P<0,05 |
| Extrait Trip | 25μg/ml | 0 | NS |
| Extraits myrte + Trip | 10 et 25 μg/ml | 60,3 | P<0,01 |
| Mb : membranes ; Trip : *Tripterygium wilfordii* ; NS : non significatif. | | | |

Sans stimulation, les cellules dendritiques dérivées des monocytes ne produisent pas d'IL-6. Cependant, les membranes de *C. acnes* induisent significativement la production d'IL-6 par les cellules dendritiques dérivées des monocytes. Le contrôle positif, la dexamethasone à 300 ng/ml, inhibe de façon significative cette production, ces résultats attendus permettent de valider ce test.

L'extrait de myrte à 10 μg/ml inhibe significativement la production d'IL-6 par les cellules dendritiques dérivées des monocytes. L'extrait de *Tripterygium wilfordii* à 25 μg/ml ne modifie pas la libération d'IL-6 par rapport à la condition de stimulation par les membranes de *C. acnes.* En revanche, l'association des extraits de myrte et de *Tripterygium wilfordii* aux mêmes concentrations que précédemment inhibe fortement et de manière significative (p<0,01) la production d'IL-6 avec un effet synergique (Tableau 1). En effet, en présence de ces 2 extraits, l'inhibition de la production d'IL-6 atteint 60%, contre 42% si on additionne les effets des extraits utilisés individuellement.

Les résultats sur l'inhibition de production d'interleukines 8 (IL-8) par des cellules dendritiques dérivées des monocytes, stimulées avec les membranes de *C. acnes* sont présentés dans le tableau 2 ci-dessous.

[Tableau 2]

| Produits | Concentration | Inhibition (%) | Statistiques |
|---|---|---|---|
| Contrôle (Mb *C. acnes*) | - | 0 | - |

(suite)

| Produits | Concentration | Inhibition (%) | Statistiques |
|---|---|---|---|
| Dexamethasone | 300 ng/ml | 47,6 | P<0,05 |
| Extrait de myrte | 10 µg/ml | 22,5 | NS |
| Extrait Trip | 25µg/ml | 0 | NS |
| Extraits myrte + Trip | 10 et 25 µg/ml | 40,8 | P<0,05 |
| Mb : membranes ; Trip : *Tripterygium wilfordii* ; NS : non significatif. | | | |

Sans stimulation, les cellules dendritiques dérivées des monocytes ne produisent pas d'IL-8. Cependant, les membranes de *C. acnes* induisent significativement la production d'IL-8 par les cellules dendritiques dérivées des monocytes. Le contrôle positif, la dexamethasone à 300 ng/ml, inhibe de façon significative cette production, ces résultats attendus permettent de valider ce test.

L'extrait de myrte à 10 µg/ml seul ne suffit pas pour inhiber la production d'IL-8 par les cellules dendritiques dérivées des monocytes. De façon similaire, l'extrait de *Tripterygium wilfordii à* 25 µg/ml seul, ne modifie pas la libération d'IL-8 par rapport à la condition de stimulation par les membranes de *C. acnes.* En revanche, l'association des extraits de myrte et de *Tripterygium wilfordii* aux mêmes concentrations inhibe de manière significative ($p<0,05$) la production d'IL-8 démasquant un effet synergique (Tableau 2).

Les résultats sur l'inhibition de production d'interleukines 10 (IL-10) par des cellules dendritiques dérivées des monocytes, stimulées avec les membranes de *C. acnes* sont présentés dans le tableau 3 ci-dessous.

[Tableau 3]

| Produits | Concentration | Inhibition (%) | Statistiques |
|---|---|---|---|
| Contrôle (Mb *C. acnes*) | - | 0 | - |
| Dexamethasone | 300 ng/ml | 41,7 | P<0,01 |
| Extrait de myrte | 10 µg/ml | 42,5 | P<0,01 |
| Extrait Trip | 25µg/ml | 2,7 | NS |
| Extraits myrte + Trip | 10 et 25 µg/ml | 67,2 | P<0,001 |
| Mb : membranes ; Trip : *Tripterygium wilfordii* ; NS : non significatif. | | | |

Sans stimulation, les cellules dendritiques dérivées des monocytes ne produisent pas d'IL-10. Cependant, les membranes de *C. acnes* induisent significativement la production d'IL-10 par les cellules dendritiques dérivées des monocytes. Le contrôle positif, la dexamethasone à 300 ng/ml, inhibe de façon significative cette production, ces résultats attendus permettent de valider ce test.

L'extrait de myrte à 10 µg/ml inhibe significativement la production d'IL-10 par les cellules dendritiques dérivées des monocytes. L'extrait de *Tripterygium wilfordii* à 25 µg/ml ne modifie pas la libération d'IL-10 par rapport à la condition de stimulation par les membranes de *C. acnes.* En revanche, l'association des extraits de myrte et de *Tripterygium wilfordii* aux mêmes concentrations que précédemment inhibe fortement et de manière très significative ($p<0,001$) la production d'IL-10 avec un effet synergique (Tableau 3). En effet, en présence de ces 2 extraits la production d'IL-10 est diminuée de plus de 2/3, contre moins de la moitié si on additionne les effets des extraits utilisés individuellement.

Les résultats sur l'inhibition de production d'interleukines 12p40 (IL-12p40) par des cellules dendritiques dérivées des monocytes, stimulées avec les membranes de *C. acnes* sont présentés dans le tableau 4 ci-dessous.

[Tableau 4]

| Produits | Concentration | Inhibition (%) | Statistiques |
|---|---|---|---|
| Contrôle (Mb *C. acnes*) | - | 0 | - |
| Dexamethasone | 300 ng/ml | 79,5 | P<0,001 |
| Extrait de myrte | 10 µg/ml | 48,3 | P<0,01 |
| Extrait Trip | 25µg/ml | 9,6 | NS |

(suite)

| Produits | Concentration | Inhibition (%) | Statistiques |
|---|---|---|---|
| Extraits myrte + Trip | 10 et 25 µg/ml | 70,7 | P<0,001 |
| Mb : membranes ; Trip : *Tripterygium wilfordii* ; NS : non significatif. | | | |

Sans stimulation, les cellules dendritiques dérivées des monocytes ne produisent pas d'IL-12p40. Cependant, les membranes de *C. acnes* induisent significativement la production d'IL-12p40 par les cellules dendritiques dérivées des monocytes. Le contrôle positif, la dexamethasone à 300 ng/ml, inhibe de façon significative cette production, ces résultats attendus permettent de valider ce test.

L'extrait de myrte à 10 µg/ml inhibe significativement la production d'IL-12p40 par les cellules dendritiques dérivées des monocytes, de près de 50%. L'extrait de *Tripterygium wilfordii* à 25 µg/ml seul, tend à diminuer la libération d'IL-12p40, mais sans atteindre la significativité, par rapport à la condition de stimulation par les membranes de *C. acnes.* En revanche, l'association des extraits de myrte et de *Tripterygium wilfordii* aux mêmes concentrations inhibe de manière très significative (p<0,001) la production d'IL-12p40 et de façon synergique (Tableau 4).

*Stimulation par un biofilm de C. acnes*

Les résultats sur la production d'IL-10 par des cellules dendritiques dérivées des monocytes, stimulées avec un biofilm de *C. acnes* sont présentés dans le tableau 5 ci-dessous.

[Tableau 5]

| Produits | Concentration | Inhibition (%) | Statistiques |
|---|---|---|---|
| Contrôle (Bf *C. acnes*) | - | 0 | - |
| Dexamethasone | 300 ng/ml | 38,6 | P<0,05 |
| Extraits myrte + Trip | 10 et 25 µg/ml | 59,8 | P<0,01 |
| Bf : biofilm ; Trip : *Tripterygium wilfordii* ; NS : non significatif. | | | |

Sans stimulation, les cellules dendritiques dérivées des monocytes ne produisent pas d'IL-10. Cependant, les biofilms de C. *acnes* induisent significativement la production d'IL-10 par les cellules dendritiques dérivées des monocytes. Le contrôle positif, la dexamethasone à 300 ng/ml, inhibe de façon significative cette production, ces résultats attendus permettent de valider ce test.

L'association des extraits de myrte (10 µg/ml) et de *Tripterygium wilfordii* (25 µg/ml) inhibe encore plus fortement (p<0,01) que la dexamethasone la production d'IL-10 (Tableau 5).

En conclusion, les inventeurs mettent en évidence que, pris isolément, les extraits de myrte et de *Tripterygium wilfordii* présentent une activité antiinflammatoire relativement modeste dans ces modèles en inhibant les productions de différentes interleukines. En revanche, les inventeurs démontrent une synergie d'action pour l'activité antiinflammatoire, en associant ces deux extraits.

## Revendications

1. Association comprenant un extrait de myrte et un extrait de Tripterygium wilfordii, dans laquelle l'extrait de myrte est une fraction apolaire comprenant des myrtucommulones et de l'acide ursolique et l'extrait de *Tripterygium wilfordii* comprend au moins un triterpène pentacyclique choisi parmi la Tingénine A, la Tingénine B, le Célastrol, et leurs mélanges.

2. Association selon la revendication 1, **caractérisée en ce que** les myrtucommulones comprennent les myrtucommulones A, B', D, B, l'isosemimyrtucommulone et la semimyrtucommulone.

3. Association selon la revendication 1 ou 2, **caractérisée en ce que** la teneur en myrtucommulones totales est comprise entre 3% et 10% en poids, par rapport au poids total de l'extrait sec de myrte.

4. Association selon l'une quelconque des revendications 1 à 3, **caractérisée en ce que** la teneur en acide ursolique est

comprise entre 10% et 30% en poids, par rapport au poids total de l'extrait sec de myrte.

**5.** Association selon l'une quelconque des revendications 1 à 4, **caractérisée en ce que** l'extrait de *Tripterygium wilfordii* comprend de la Tingénine A, de la Tingénine B, et du Célastrol.

**6.** Association selon l'une quelconque des revendications 1 à 5, **caractérisée en ce que** l'extrait de *Tripterygium wilfordii* est susceptible d'être obtenu par le procédé suivant :

(i) Une phase de prolifération de cellules de *Tripterygium wilfordii* dans un milieu de prolifération,
(ii) Une phase d'élicitation par ajout d'un cocktail d'élicitation à la culture cellulaire obtenue à l'étape (i), ledit cocktail d'élicitation comprenant au moins un éliciteur de type composé monocarboxylique et au moins un éliciteur biotique, et
(iii) La préparation d'un extrait comprenant des triterpènes pentacycliques à partir de la culture cellulaire obtenue à l'étape (ii).

**7.** Association selon l'une quelconque des revendications 1 à 6, **caractérisée en ce que** l'extrait de *Tripterygium wilfordii* comprend 90% en poids du au moins un triterpène pentacyclique par rapport au poids total de l'extrait sec.

**8.** Association selon l'une quelconque des revendications 1 à 7, **caractérisée en ce que** l'extrait de *Tripterygium wilfordii* comprend entre 1% et 20% de Tingénine A en poids par rapport au poids total de l'extrait sec, entre 1% et 20% de Tingénine B en poids par rapport au poids de l'extrait sec et au moins 60% de Célastrol en poids par rapport au poids total de l'extrait sec.

**9.** Association selon l'une quelconque des revendications 1 à 8, pour son utilisation dans le traitement de l'inflammation induite par C. *acnes.*

**10.** Association selon l'une quelconque des revendications 1 à 8, pour son utilisation dans le traitement d'une peau acnéique.

**11.** Composition cosmétique ou dermatologique comprenant une association selon l'une quelconque des revendications 1 à 8 avec au moins un excipient cosmétiquement ou dermatologiquement acceptable.

**12.** Composition selon la revendication 11, **caractérisée en ce qu'**elle comprend 0,01% à 1% d'extrait sec de *Tripterygium wilfordii* en poids par rapport au poids total de la composition.

**13.** Composition selon la revendication 11 ou 12, **caractérisée en ce qu'**elle comprend 0,01% à 1%, en poids d'un extrait sec de myrte par rapport au poids total de la composition.

**14.** Composition selon l'une quelconque des revendications 11 à 13, **caractérisée en ce qu'**elle se présente sous une forme adaptée à une application topique.

**15.** Composition selon l'une quelconque des revendications 11 à 14, pour son utilisation dans le traitement de l'inflammation induite par *C. acnes.*

**16.** Composition selon l'une quelconque des revendications 11 à 14, pour son utilisation dans le traitement d'une peau acnéique.

**Patentansprüche**

**1.** Kombination, umfassend einen Myrtenextrakt und einen *Tripterygium wilfordii*-Extrakt, wobei der Myrtenextrakt eine unpolare Fraktion ist, die Myrtucommulone und Ursolsäure enthält und der *Tripterygium wilfordii*-Extrakt mindestens ein pentacyclisches Triterpen umfasst, das aus Tingenin A, Tingenin B, Celastrol und deren Gemischen ausgewählt ist.

**2.** Kombination nach Anspruch 1, **dadurch gekennzeichnet, dass** die Myrtucommulone die Myrtucommulone A, B', D, B, Isosemimyrtucommulon und Semimyrtucommulon umfassen.

3. Kombination nach Anspruch 1 oder 2, **dadurch gekennzeichnet, dass** der Gehalt an Myrtucommulonen insgesamt zwischen 3 und 10 Gew.-%, bezogen auf das Gesamtgewicht des Myrtentrockenextrakts, liegt.

4. Kombination nach einem der Ansprüche 1 bis 3, **dadurch gekennzeichnet, dass** der Gehalt an Ursolsäure zwischen 10 und 30 Gew.-%, bezogen auf das Gesamtgewicht des Myrtentrockenextrakts, liegt.

5. Kombination nach einem der Ansprüche 1 bis 4, **dadurch gekennzeichnet, dass** der *Tripterygium wilfordii*-Extrakt Tingenin A, Tingenin B und Celastrol umfasst.

6. Kombination nach einem der Ansprüche 1 bis 5, **dadurch gekennzeichnet, dass** der *Tripterygium wilfordii*-Extrakt durch das folgende Verfahren erhaltbar ist:

(i) eine Phase der Vermehrung von *Tripterygium wilfordii*-Zellen in einem Vermehrungsmedium,
(ii) eine Phase der Elicitation durch Zugabe eines Elicitations-Cocktails zu der in Schritt (i) erhaltenen Zellkultur, wobei der Elicitations-Cocktail mindestens einen Elicitor vom Typ einer Monocarbonsäureverbindung und mindestens einen biotischen Elicitor umfasst, und
(iii) die Herstellung eines Extrakts, der pentacyclische Triterpene umfasst, aus der in Schritt (ii) erhaltenen Zellkultur.

7. Kombination nach einem der Ansprüche 1 bis 6, **dadurch gekennzeichnet, dass** der *Tripterygium wilfordii*-Extrakt 90 Gew.-% des mindestens einen pentazyklischen Triterpens, bezogen auf das Gesamtgewicht des Trockenextrakts, umfasst.

8. Kombination nach einem der Ansprüche 1 bis 7, **dadurch gekennzeichnet, dass** der *Tripterygium wilfordii*-Extrakt zwischen 1 und 20 Gew.-% Tingenin A, bezogen auf das Gesamtgewicht des Trockenextrakts, zwischen 1 und 20 Gew.-% Tingenin B, bezogen auf das Gewicht des Trockenextrakts, und mindestens 60 Gew.-% Celastrol, bezogen auf das Gesamtgewicht des Trockenextrakts, umfasst.

9. Kombination nach einem der Ansprüche 1 bis 8 zu ihrer Verwendung bei der Behandlung von durch *C. acnes* verursachten Entzündungen.

10. Kombination nach einem der Ansprüche 1 bis 8 zu ihrer Verwendung bei der Behandlung von Akne-Haut.

11. Kosmetische oder dermatologische Zusammensetzung, die eine Kombination nach einem der Ansprüche 1 bis 8 mit mindestens einem kosmetisch oder dermatologisch akzeptablen Trägerstoff umfasst.

12. Zusammensetzung nach Anspruch 11, **dadurch gekennzeichnet, dass** sie 0,01 bis 1 Gew.-% Trockenextrakt aus *Tripterygium wilfordii,* bezogen auf das Gesamtgewicht der Zusammensetzung, umfasst.

13. Zusammensetzung nach Anspruch 11 oder 12, **dadurch gekennzeichnet, dass** sie 0,01 bis 1 Gew.-% eines Myrtentrockenextrakts, bezogen auf das Gesamtgewicht der Zusammensetzung, umfasst.

14. Zusammensetzung nach einem der Ansprüche 11 bis 13, **dadurch gekennzeichnet, dass** sie in einer für eine topische Anwendung geeigneten Form vorliegt.

15. Zusammensetzung nach einem der Ansprüche 11 bis 14 zu ihrer Verwendung bei der Behandlung von durch *C. acnes* verursachten Entzündungen.

16. Zusammensetzung nach einem der Ansprüche 11 bis 14 zu ihrer Verwendung bei der Behandlung von Akne-Haut.

## Claims

1. An association comprising a myrtle extract and an extract of Tripterygium wilfordii., wherein the myrtle extract is a nonpolar fraction comprising myrtucommulones and ursolic acid, and the Tripterygium wilfordii extract comprises at least one pentacyclic triterpene selected from Tingenin A, Tingenin B, Celastrol, and mixtures thereof.

2. The association according to claim 1, **characterised in that** the myrtucommulones comprise myrtucommulones A,

B', D, B, isosemimyrtucommulone and semimyrtucommulone.

3. The association according to claim 1 or 2, **characterised in that** the content of total myrtucommulones is comprised between 3% and 10% by weight, relative to the total weight of the dry myrtle extract.

4. The association according to any one of claims 1 to 3, **characterised in that** the content of ursolic acid is between 10% and 30% by weight, relative to the total weight of the dry myrtle extract.

5. The association according to any of claims 1 to 4, **characterized in that** the *Tripterygium wilfordii* extract comprises Tingenin A, Tingenin B, and Celastrol.

6. The association according to any one of claims 1 to 5, **characterised in that** the extract of *Tripterygium wilfordii* is obtainable by the following method:

(i) a proliferation phase of *Tripterygium wilfordii* cells in a proliferation medium,
(ii) an elicitation phase by addition of an elicitation cocktail to the cell culture obtained in step (i), said elicitation cocktail comprising at least one monocarboxylic compound elicitor and at least one biotic elicitor, and
(iii) the preparation of an extract comprising pentacyclic triterpenes from the cell culture obtained in step (ii).

7. The association according to any one of claims 1 to 6, **characterised in that** the extract of *Tripterygium wilfordii* comprises 90% by weight of at least one pentacyclic triterpene relative to the total weight of the dry extract.

8. The association according to any one of claims 1 to 7, **characterised in that** the extract of *Tripterygium wilfordii* comprises between 1% and 20% tingenin A by weight, relative to the total weight of the dry extract, between 1% and 20% tingenin B by weight relative to the weight of the dry extract and at least 60% celastrol by weight relative to the total weight of the dry extract.

9. The association according to any one of claims 1 to 8, for its use in the treatment of inflammation induced by *C. acnes.*

10. The association according to any one of claims 1 to 8, for its use in the treatment of acne-prone skin.

11. A cosmetic or dermatological composition comprising an association according to any one of claims 1 to 8 with at least one cosmetically or dermatologically acceptable excipient.

12. The composition according to claim 11, **characterised in that** it comprises 0.01% to 1% dry extract of *Tripterygium wilfordii* by weight relative to the total weight of the composition.

13. The composition according to claim 11 or 12, **characterised in that** it comprises 0.01% to 1%, by weight of a dry myrtle extract relative to the total weight of the composition.

14. The composition according to any one of claims 11 to 13, **characterised in that** it presents itself in a form suitable for a topical application.

15. The composition according to any one of claims 11 to 14, for its use in the treatment of inflammation induced by *C. acnes.*

16. The composition according to any one of claims 11 to 14, for its use in the treatment acne-prone skin.

Figure 1

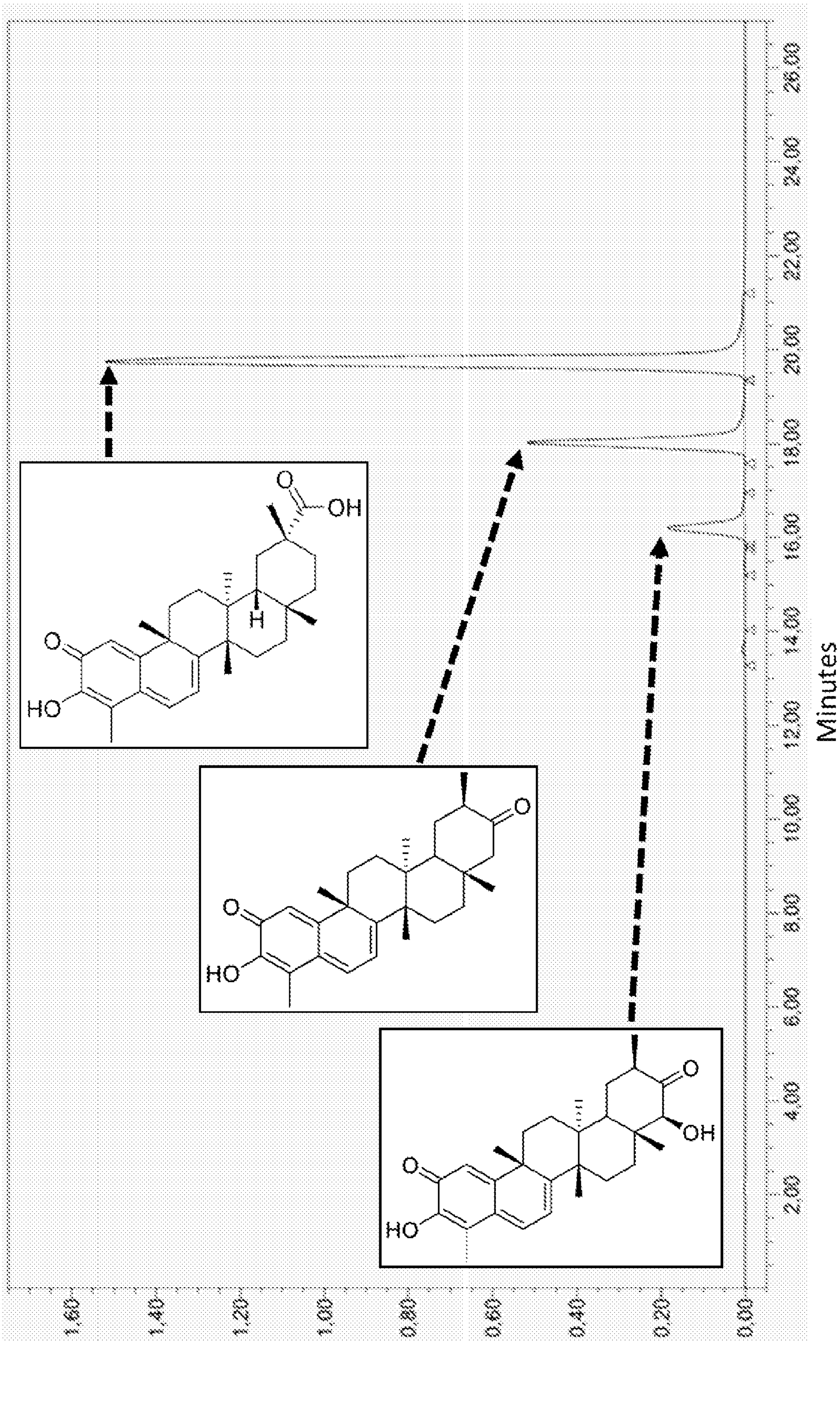
Minutes
2.00
4.00
6.00
8.00
10.00
12.00
14.00
16.00
18.00
20.00
22.00
24.00
26.00
0.00
0.20
0.40
0.60
0.80
1.00
1.20
1.40
1.60

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 1112079 A
- FR 2992862
- EP 3454875 A


### Littérature non-brevet citée dans la description


- **HOLMBERG et al.** *Clin. Microbiol. Infect.*, 2009, vol. 15, 787-795
- **CRAIG et al.** *J. Am. Acad. Dermatol.*, 2007, 722-724
- **DAGNELIE et al.** *Journal of the European Academy of Dermatology*, 2019, vol. 33 (12), 2340-2348
- **COPPEDE et al.** *Plant Cell Tiss Organ Cult*, 2017, vol. 118, 33-43
- **MURASHIGE ; SKOOG**. *Physiologia Plantarum*, 1962, vol. 15, 473-497